(19) Europäisches Patentamt

European Patent Office

Office européen des brevets

(11) EP 0 639 347 B1

(12) EUROPEAN PATENT SPECIFICATION

(45) Date of publication and mention
of the grant of the patent:
06.06.2001 Bulletin 2001/23

(21) Application number: 93901550.9

(22) Date of filing: 13.01.1993

(51) Int Cl.7: A61B 8/00, G01S 7/52

(86) International application number:
PCT/JP93/00042

(87) International publication number:
WO 93/13710 (22.07.1993 Gazette 1993/18)

(54) DIGITAL PHASE SHIFTER

DIGITALER PHASENSCHIEBER

DEPHASEUR NUMERIQUE

(84) Designated Contracting States:
DE FR GB NL

(30) Priority: 14.01.1992 JP 471392

(43) Date of publication of application:
22.02.1995 Bulletin 1995/08

(73) Proprietor: YOKOGAWA MEDICAL SYSTEMS,
LTD
Hino-shi, Tokyo 191 (JP)

(72) Inventors:
• AMEMIYA, Shinichi
Tokyo, 192-03 (JP)
• SUZUKI, Yoichi
Tachikawa-Shi, Tokyo, 190 (JP)

(74) Representative: Henkel, Feiler, Hänzel
Möhlstrasse 37
81675 München (DE)

(56) References cited:
JP-A- 1 076 839          JP-A- 61 075 685
JP-A- 63 222 745         US-A- 3 997 772
US-A- 3 997 773

• ELECTRONICS LETTERS, vol. 26, no. 19, 13
September 1990 pages 1608-1610, XP 000106903
LIU G -S ET AL 'PROGRAMMABLE FRACTIONAL
SAMPLE DELAY FILTER WITH LAGRANGE
INTERPOLATION'

## Description

(Technical Field)

The present invention relates to a digital phase shifting apparatus. To put it in more detail, the present invention relates to a digital phase shifting apparatus which is capable of shifting the phase of digital data by a fine step shorter than the sampling period of an A/D converter. The digital phase shifting apparatus is implemented as delay circuits and useful typically for an ultrasonic diagnosing apparatus.

(Background Art)

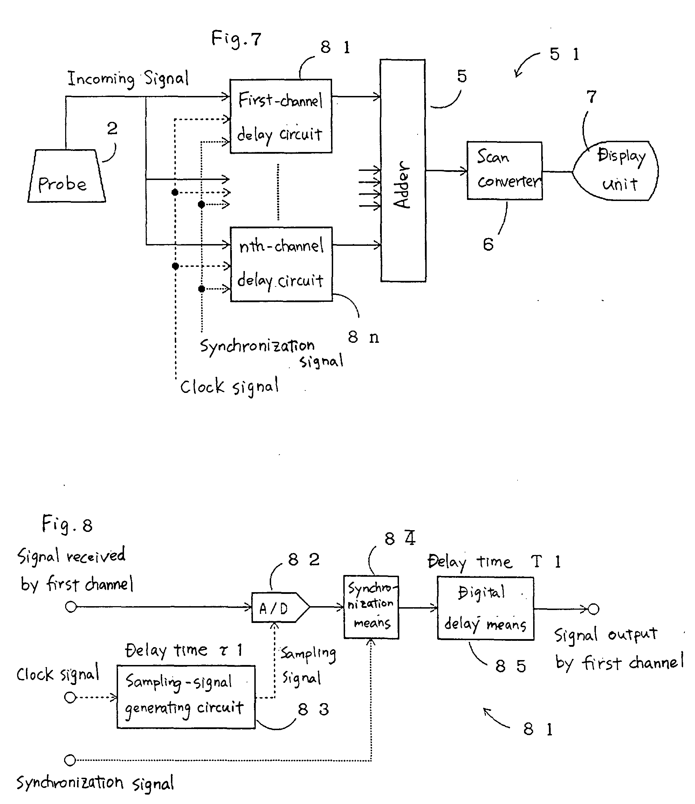

An example of the conventional digital phase shifting apparatus which is capable of shifting the phase of digital data by a fine step shorter than the sampling period of an A/D converter is disclosed in Japanese Patent Laid-open No. 63-222745. A block diagram showing the configuration of an ultrasonic diagnosing apparatus disclosed in Japanese Patent Laid-open No. 63-222745 is shown in Fig. 7.

In this ultrasonic diagnosing apparatus 51, a signal received from a probe 2 undergoes phase adjustment for all channels by a number of delay circuits (or phase shifting circuits): a first-channel delay circuit 81 to an $n$th-channel delay circuit 8n. The outputs of the delay circuits are then summed up by an adder 5. The adder 5 carries out a beam forming process for generating a sound-ray reception signal. Based on all the sound-ray reception signals, a scan converter 6 produces pictures to be displayed on a display apparatus 7.

A detailed block diagram of the first-channel delay circuit 81 is shown in Fig. 8. The delay circuits of the other channels each have entirely the same configuration. A signal received by the first-channel delay circuit 81 is supplied to an A/D converter 82. A clock signal having a predetermined period $\Delta T$ is fed to a sampling signal delay means 83. A synchronization signal also having the predetermined period $\Delta T$ is supplied to a synchronization means 84. The synchronization means 83 delays the clock signal by a time rl shorter than the period $\Delta T$, producing a sampling signal output to the A/D converter 82.

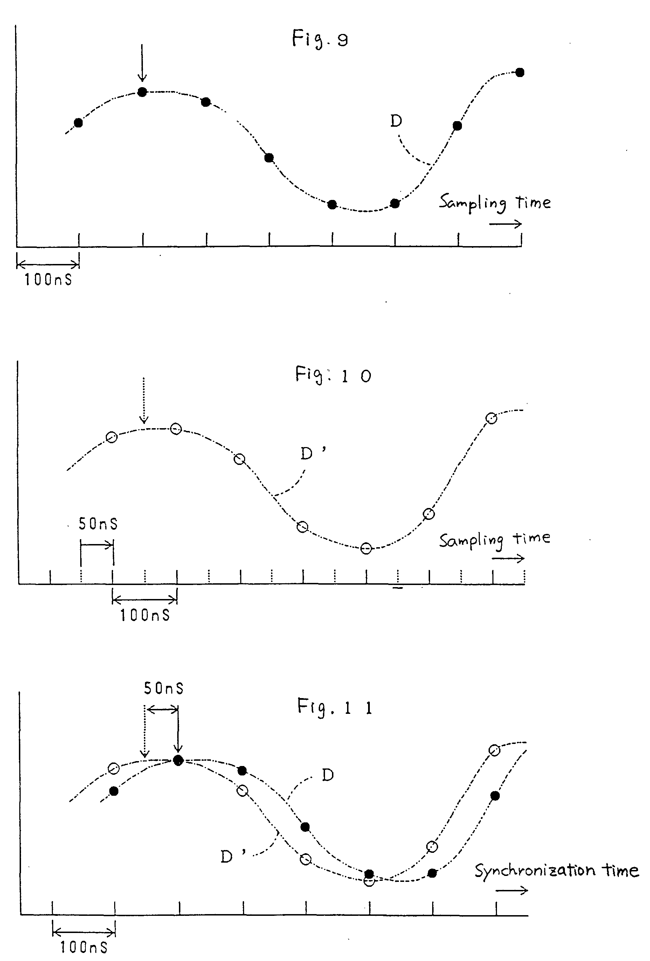

The A/D converter 82 samples the signal received by the first-channel delay circuit 81 at sampling intervals equal to $\Delta T$ by using the sampling signal, converting the received signal into digital data. The signal received by the first-channel delay circuit 81 is denoted by notations D and D' in Figs. 9 and 10 respectively. Digital data obtained by using sampling signals with delay times different from each other is, on the other hand, denoted by symbols ● and ○ in Figs. 9 and 10 respectively. For a sampling interval $\Delta T$ of 100 ns, the symbols ● and ○ denote sampled digital data for $\tau 1$ equal to 0 and 50 ns respectively.

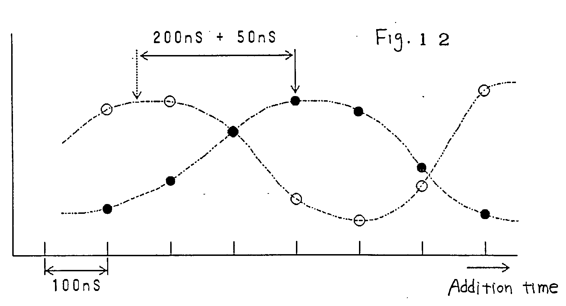

The synchronization means 84 is used for synchronization. For example, a train of pieces of digital data denoted by the symbol ○ lags behind a train of pieces of digital data ● due to the synchronization by $\tau 1$, which is shorter than the sampling period, as shown in Fig. 11. A digital delay means 85 is used for further delaying a signal output by the synchronization means 84 by a delay time T1 which is equal to a multiple of the sampling interval $\Delta T$. As a result, the first-channel delay circuit 81 outputs digital data lagging behind the signal received by the first channel by ($\tau 1 + T1$).

A case with T1 and $\tau 1$ having values of 200 ns and 50 ns respectively is shown in Fig. 12. As a whole, the train of pieces of digital data denoted by the symbol ○ lags behind the train of pieces of digital data denoted by the symbol ● by a total of 250 ns.

In the conventional delay circuits 81 to 8n described above, however, sampling signals to shift the phase by amounts of time slightly different from channel to channel must inevitably be used, giving rise to a problem that the control becomes complicated.

US-A-3 997 773 discloses a digital phase shifting apparatus according to the pre-characterizing part of the claim 1.

A further digital phase shifter is disclosed in US-A-3 997 772.

It is an object of the present invention to provide an improved digital phase shifting apparatus which is suitable for a real time system.

The above object is achieved by a digital phase shifting apparatus according to claim 1. The dependent claims related to further advantages as packed to the present invention.

(Brief Description of the Drawings)

Fig. 1 is a block diagram of an ultrasonic diagnosing apparatus provided by the present invention;
Fig. 2 is a block diagram of an embodiment implementing a delay circuit in accordance with the present invention;
Fig. 3 is an explanatory diagram used for describing interpolated data;
Fig. 4 is an explanatory diagram used for describing a train of pieces of digital data obtained by interpolation;
Fig. 5 is an explanatory diagram used for describing a state in which a delay time shorter than a sampling interval is resulted in;
Fig. 6 is an explanatory diagram used for describing a state in which a long delay time is resulted in as a whole;
Fig. 7 is an explanatory diagram used for describing the conventional ultrasonic diagnosing apparatus;
Fig. 8 is a block diagram of the conventional delay circuit;
Fig. 9 is an explanatory diagram used for describing digital data produced by a sampling signal without delay;

Fig. 10 is an explanatory diagram used for describing digital data produced by sampling signals with delays;

Fig. 11 is an explanatory diagram used for describing a state in which a delay time shorter than a sampling interval is resulted in; and

Fig. 12 is an explanatory diagram used for describing a state in which a long delay time is resulted in as a whole.

(Description of the Preferred Embodiment)

The present invention will become apparent from the following detailed description of a preferred embodiment with reference to accompanying diagrams. It should be noted, however, that the range of the present invention is not limited to the preferred embodiment.

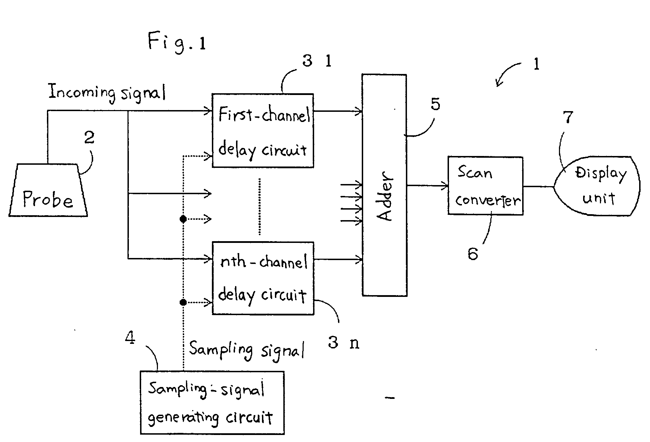

A block diagram of an ultrasonic diagnosing apparatus 1 employing a digital phase shifting apparatus provided by the present invention is shown in Fig. 1.

In the ultrasonic diagnosing apparatus 1, signals for n channels received by a probe 2 undergo phase adjustment for the individual channels in a number of delay circuits (or phase shifting circuits): a first-channel delay circuit 31 to an $n$th-channel delay circuit 3n. Outputs of the delay circuits are summed up by an adder 5. The adder 5 then carries out a beam forming process to produce a sound-ray signal. Based on all the sound-ray signals, a scan converter 6 generates pictures to be displayed on a display unit 7.

The delay circuits 31 to 3n are provided with a common sampling signal generated by a sampling-signal generating circuit 4.

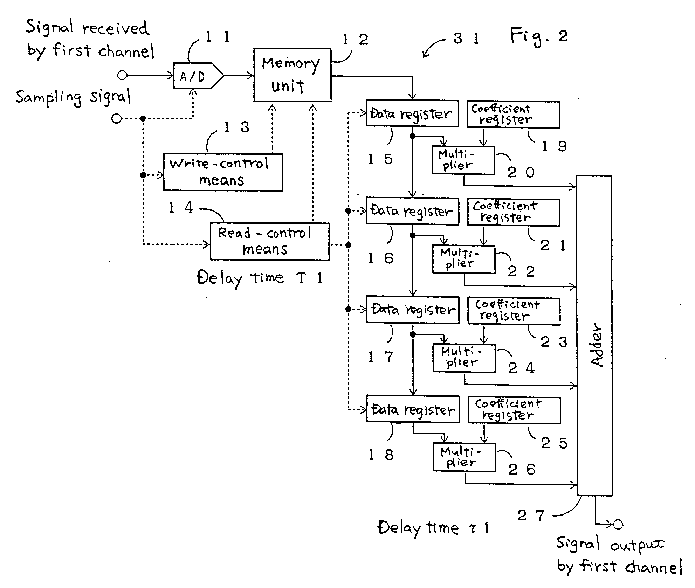

A detailed block diagram of the first-channel delay circuit 31 is shown in Fig. 2. The delay circuits of the other channels have exactly the same configuration. An analog signal received by the first channel is supplied to an A/D converter 11. A sampling signal having a period $\Delta T$ is fed to the A/D converter 11, a write-control means 13 and a read-control means 14.

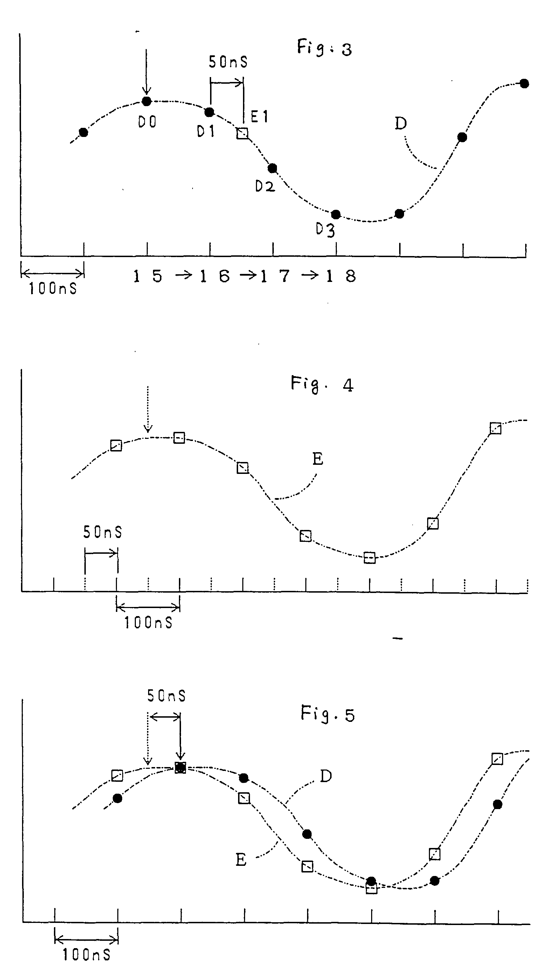

Driven by the sampling signal, the A/D converter 11 samples the signal received by the first channel at sampling intervals each equal to the period $\Delta T$. In Fig. 3, the analog signal received by the first channel is indicated by a notation D whereas sampled digital data is denoted by a symbol ●. A case with a sampling interval $\Delta T$ of 100 ns is shown in Fig. 3. The sampled digital data is stored in a memory unit 12 at an address specified by the write-control means 13. The memory unit 12 outputs data stored at an address specified by the read-control means 14. A difference between addresses specified by the write-control means 13 and the read-control means 14 corresponds to a desired multiple of the sampling interval $\Delta T$. These addresses are updated synchronously with the sampling signal.

A data register 15 is used for storing a piece of digital data read out from the memory unit 12. Each time a new piece of digital data is stored into the data register 15, a piece of digital data stored previously in the data register 15 is shifted to a data register 16. At the same time, pieces of data are shifted from the data register 16 to a data register 17, and from the data register 17 to a data register 18. In this way, four pieces of digital data sampled at points of time separated from each other by the sampling interval $\Delta T$ are stored in the data registers 15 to 18. The four pieces of data are updated by the shifting operations synchronously with the sampling signal. The read-control means 14 controls operations to read data from the memory unit 12 in such a way that four pieces of data stored in the data registers 15 to 18 are ahead of the current sampling time by (T1 - $\Delta T$), T1, (T1 + $\Delta T$) and (T1 + 2$\Delta T$) respectively.

Data with a fine delay time rl shorter than the sampling time $\Delta T$ is formed from the four pieces of data typically by means of mixed spline interpolation to be described later. Coefficient registers 19, 21, 23 and 25 are each used for storing an interpolation coefficient for use in the mixed spline interpolation. Multipliers 20, 22, 24 and 26 are used for multiplying the digital data stored in the data registers 15, 16, 17 and 18 by the interpolation coefficients of the mixed spline interpolation stored in the coefficient registers 19, 21, 23 and 25, respectively. The products are output to an adder 27 to be summed up. The operations to process such data and generate the output are carried out synchronously with the sampling signal.

A concept of computing interpolated digital data E1 by the mixed spline interpolation from four pieces of digital data D0 to D3 stored in the data registers 15 to 18 respectively is shown in Fig. 3. The figure shows a case with $\Delta T$ = 100 ns and $\tau1$ = 50 ns.

For example, let the digital data E1 be data interpolated between the data D1 and D2 and the distance from the data E1 to a point of interpolation be u$\Delta T$. According to a formula of the mixed spline interpolation, the E1 is given by the following equation:

$$E1 = -(1/6)u(u-1)(u-2)D0 + (1/2)(u+1)(u-1)(u-2)D1 -$$

$$(1/2)(u+1)u(u-2)D2 + (1/6)(u+1)u(u-1)D3$$

It is obvious from the above equation that the interpolated data E1 is a sum of the four pieces of data D0 to D1 with each piece of data multiplied by a predetermined weight coefficient. The coefficients are univocally determined by a relative distance u from the data D1 to the point of interpolation. For $\tau1$ = 50 ns, the relative distance u is 0.5. Coefficients for a desired point of interpolation are stored in the coefficient registers 19, 21, 23 and 25 in advance to be used in the addition by the adder 27 to give the desired interpolated data E1.

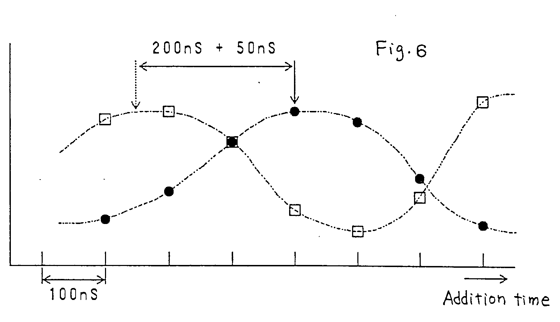

A signal output by the first channel is shown in Fig. 4 as a time-axis series of pieces of digital data each denoted by a symbol □. The output signal of the first channel is digital data lagging behind the digital data stored in the data register 16 by 50 ns as shown in Fig. 5. It should be noted that the amount of delay τ1 is not limited to the value 50 ns. The amount of delay τ1 can be determined arbitrarily by selecting the position of the interpolation point. On the other hand, the digital data stored in the data register 16 was obtained at a sampling time ahead of the current sampling time by T1. As a result, the data E1 is digital data lagging by the sum (T1 + τ1). A typical case for T1 = 200 ns and τ1 = 50 ns is shown in Fig. 6. The case shown in Fig. 6 corresponds to that of Fig. 12.

The delay circuits of the other channels each carry out the same operations to produce a train of pieces of data lagging behind the signal input to the channel by a desired time.

A single train of sampling pulses common to the delay circuits 31 to 3n described above is used without adjusting its phase, resulting in control simpler than that of the conventional system. On top of that, stability of the delay time is enhanced. It should be noted that the use of the mixed spline interpolation allows calculation to be based on only four points before and after an interpolation point. As a result, the interpolation technique is suitable for a real-time system. On top of that, the interpolation coefficients are determined univocally for a given delay time τ1, allowing the mixed spline interpolation to be implemented by the same configuration as that of a transversal filter for example.

## Claims

1. A digital phase shifting apparatus comprising:

    means (11) for sampling analog input signals at sampling time intervals and for generating a time-axis train of sampled data therefrom;
    means (12) for storing said time-axis train of sampled data one after another; and
    means for generating phase shifted data by performing an interpolation using said sampled data which were read from said means for storing the time-axis train of sampled data;

    characterized in that
    said means for generating phase shifted data are adapted to perform the interpolation based on the following equation:

$$E_x = -(1/6)u(u-1)(u-2)D_{x-1} + (1/2)(u+1)(u-1)(u-2)D_x -$$

$$(1/2)(u+1)u(u-2)D_{x+1} - (1/6)(u+1)u(u-1)D_{x+2}$$

    wherein $E_x$ is phase shifted data interpolated between the sampled data $D_x$ and $D_{x+1}$, u being the

relative distance from the sampled data $D_x$ to the point of interpolation, and x is the (discrete) time axis.

2. A digital phase shifting apparatus of claim 1, wherein said means for generating phase shifted data includes

    means (14) for reading out data sampled at times ahead of a current sampling time by desired time differences one after another in synchronization with sampling which provides said time-axis train of sampled data,
    a plurality of register means (15-18) for holding said sampled data, said sampled data being selected from a most recently read out data to a data ahead of the most recently read out data by a predetermined data count,
    a plurality of coefficient register means (19,21,23,25) corresponding to said plurality of register means and for holding a predetermined weighted coefficient,
    a plurality of multiplied means (20,22,24,26) corresponding to said plurality of register means and to said plurality of coefficient register means, and for multiplying a sampled data from said corresponding register means with a weighted coefficient from said corresponding coefficient register means, and
    an adder means (27) for summing up outputs from said plurality of multiplier means.

3. An ultrasonic imaging apparatus comprising:

    probe means (2) for transmitting and receiving ultrasound waves;
    a plurality of said means for generating phase shifted data according to claim 1 or 2;
    another adder means (5) for summing up outputs from said plurality of adder means;
    scan converter means (6) for converting scans corresponding to outputs of said adder means into scans of an image to be displayed; and
    display means (7) for displaying said image.

## Patentansprüche

1. Digitale Phasenverschiebungsvorrichtung mit:

    einem Mittel (11) zur Abtastung analoger Eingangssignale mit Abtastzeitintervallen und zur Erzeugung einer Zeitachsenfolge abgetasteter Daten;
    einem Mittel (12) zum Speichern der Zeitachsenfolge abgetasteter Daten nacheinander; und
    Mitteln zum Erzeugen phasenverschobener Daten durch Durchführung einer Interpolation mit den abgetasteten Daten, die aus dem Mittel zum Speichern der Zeitachsenfolge abgetasteter Daten ausgelesen wurden;

dadurch gekennzeichnet, daß

die Mittel zum Erzeugen der phasenverschobenen Daten ausgestaltet sind, um die Interpolation beruhend auf der folgenden Gleichung durchzuführen:

$$Ex = -(1/6)u(u-1)(u-2)Dx-1 + (1/2)(u+1)(u-1)(u-2)Dx -$$

$$(1/2)(u+1)u(u-2)Dx+1-(1/6)(u+1)u(u-1)Dx+2$$

wobei Ex die phasenverschobenen Daten sind, die zwischen den abgetasteten Daten Dx und Dx+1 interpoliert sind;
u der relative Abstand von den abgetasteten Daten Dx zu dem Interpolationspunkt, und wobei x die (diskrete) Zeitachse ist.

2. Digitale Phasenverschiebungsvorrichtung nach Anspruch 1, bei der die Mittel zum Erzeugen phasenverschobener Daten enthalten:

ein Mittel (14), um die abgetasteten Daten um gegebene Zeitdifferenzen zeitlich im voraus gegenüber der laufenden Abtastzeit nacheinander synchron mit der Abtastung, welche die Zeitachsenfolge der abgetasteten Daten liefert, auszulesen,
eine Mehrzahl Registermittel (15-18), um die abgetasteten Daten zu halten; wobei die abgetasteten Daten ausgewählt werden von den jüngsten ausgelesenen Daten zu Daten, die vor den jüngsten ausgelesenen Daten um einen vorgegebenen Datenzielwert voraus sind,
eine Mehrzahl Koeffizientenregistermittel (19, 21, 23, 25) entsprechend der Mehrzahlregistermittel, um einen vorgegebenen Gewichtungskoeffizienten zu halten,
einer Mehrzahl Multipliziermittel (20, 22, 24, 26) entsprechend der Mehrzahl Registermittel und der Mehrzahl Koeffizientenregistermittel, um die abgetasteten Daten aus dem entsprechenden Registermittel mit einem Gewichtungskoeffizienten aus dem entsprechenden Koeffizientenregistermittel zu multiplizieren, und
einem Addierermittel (27), um die Ausgaben der Mehrzahlmultiplizierermittel auf zu summieren..

3. Ultraschallbildgebungsvorrichtung mit:

einem Sondenmittel (2) zum Übertragen und Empfangen von Ultraschallwellen,
einer Mehrzahl der Mittel zum Erzeugen der phasenverschobenen Daten nach Anspruch 1 oder 2,
einem weiteren Addierermittel (5) zum Aufsummieren der Ausgaben der Mehrzahladdierermittel,
einem Abtastwandlermittel (6) zum Wandeln der Abtastungen entsprechend den Ausgaben des Addierermittels in Abtastungen eines anzuzeigenden Bildes, und
Anzeigemittel (7) zum Anzeigen des Bildes.

**Revendications**

1. Dispositif de déphasage numérique comprenant :

des moyens (11) pour échantillonner des signaux d'entrée analogiques à des intervalles d'instant d'échantillonnage et pour produire un train de données échantillonnées à axe temporel à partir de ces derniers ;
des moyens (12) pour stocker ledit train de données échantillonnées à axe temporel les unes derrière les autres ; et
des moyens pour produire des données déphasées en effectuant une interpolation en utilisant lesdites données échantillonnées qui sont lues à partir desdits moyens pour stocker le train de données échantillonnées à axe temporel ;

caractérisé en ce que
lesdits moyens pour produire les données déphasées sont conçus pour effectuer l'interpolation sur la base de l'équation suivante :

$$E_x = -(1/6)\, u\, (u-1)\, (u-2)\, D_{x-1} + (1/2)\, (u+1)$$

$$(u-1)\, (u-2)\, D_x - (1/2)\, (u+1)\, u\, (u-2)\, D_{x+1} -$$

$$(1/6)\, (u+1)\, u\, (u-1)\, D_{x+2}$$

dans laquelle $E_x$ est une donnée déphasée interpolée entre les données échantillonnées $D_x$ et $D_{x+1}$, u étant la distance relative de la donnée échantillonnée $D_x$ par rapport au point d'interpolation, et x est l'axe temporel (discret).

2. Dispositif de déphasage numérique selon la revendication 1, dans lequel lesdits moyens pour produire des données déphasées comprennent :

des moyens (14) pour lire des données échantillonnées à des instants en avant d'un instant d'échantillonnage courant de différences de temps souhaitées les unes après les autres en synchronisation avec un échantillonnage qui procure ledit train de données échantillonnées à axe temporel,

une pluralité de moyens de registre (15 à 18) pour maintenir lesdites données échantillonnées, lesdites données échantillonnées étant sélectionnées depuis des données lues le plus récemment jusqu'à des données en avant des données lues le plus récemment par un comptage de données prédéterminé,

une pluralité de moyens de registre de coefficient (19, 21, 23, 25) correspondant à ladite pluralité de moyens de registre et pour maintenir un coefficient pondéré prédéterminé,

une pluralité de moyens multipliés (20, 22, 24, 26) correspondant à ladite pluralité de moyens de registre et à ladite pluralité de moyens de registre de coefficient, et pour multiplier des données échantillonnées en provenance desdits moyens de registre correspondants avec un coefficient pondéré en provenance desdits moyens de registre de coefficient correspondants, et

des moyens additionneurs (27) pour sommer des sorties en provenance de ladite pluralité de moyens multiplicateurs.

3. Dispositif d'imagerie à ultrasons comprenant :

des moyens de sonde (2) pour émettre et recevoir des ondes ultrasonores ;

une pluralité desdits moyens pour produire des données déphasées selon la revendication 1 ou 2 ;

d'autres moyens additionneurs (5) pour sommer des sorties en provenance de ladite pluralité de moyens additionneurs ;

des moyens de transformation de balayage (6) pour transformer des balayages correspondant aux sorties desdits moyens additionneurs en balayages d'une image à afficher ; et

des moyens d'affichage (7) pour afficher ladite image.

Fig.1

Fig. 2

Fig. 3

50nS

D0

D1  E1

D2

D3

D

100nS   1 5 → 1 6 → 1 7 → 1 8

Fig. 4

E

50nS

100nS

50nS   Fig. 5

D

E

100nS

Fig. 6

Fig. 7

Incoming Signal

8 1

Probe 2

First-channel delay circuit

nth-channel delay circuit 8 n

Adder 5

5 1

Scan Converter 6

Display unit 7

Synchronization signal

Clock signal

Fig. 8

Signal received by first channel

Clock signal

8 2

A/D

8 4

Synchronization means

Delay time T 1

Digital delay means 8 5

Signal output by first channel

Delay time τ 1

Sampling-signal generating circuit 8 3

Sampling Signal

8 1

Synchronization signal

Fig. 9

D

Sampling Time

100nS

Fig. 10

D'

50nS

Sampling time

100nS

Fig. 11

50nS

D

D'

Synchronization time

100nS

EP 0 639 347 B1

Fig. 1 2

200nS + 50nS

100nS

Addition time

13